# DEMANDE DE BREVET EUROPEEN

(11) **EP 0 605 337 A1**
(43) Date de publication de la demande: **06.07.1994**
(21) Numéro de dépôt: 93450010.9
(22) Date de dépôt: 24.11.1993
(51) Int. Cl.: A61F 2/38

(54) **Endo-prothèse totale de genou et éléments d'essai pour pose de cette dernière**

(30) Priorité: 24.11.1992 FR 9214345
(71) Demandeur: SA PROSTEEL TECH VILLAGE VILLA SANTA FE, F-31527 Ramonville Saint Agne (FR); SOCIETE CIVILE CORUM, F-26200 Montelimar (FR)
(72) Inventeur: Lombard, Gérard, F-31500 Toulouse (FR); Jouanin, Thierry, F-26200 Moontelimar (FR)
(74) Mandataire: Ravina, Bernard

(57) **Abrégé**

L'Invention concerne une endo-prothèse totale de genou comprenant une quille (1) comportant un pied (2) destiné à être ancré dans le tibia et une tête conique (3) bloquée dans l'alésage (4) d'un plateau tibial (5) prenant appui sur la partie supérieure aplanie du tibia et recevant une pièce (6) d'insert en matière synthétique comportant des surfaces articulaires (7) sur lesquelles peut glisser en rotation une pièce condylienne (8) par l'intermédiaire des condyles externes et internes.
Le pied (2) de la qullle (1) comporte un noyau central autour duquel sont réparties deux ailettes postérieures (10) et une antérieure (11) qui, en considérant l'une quelconque section droite est moins large que les deux précédentes.

Le plateau tibial comprend un bossage (12) traversé par l'alésage (4) et deux ailettes (13) postérieures venant en regard des ailettes (10). Le bossage comprend une fente (14) de positionnement dans laquelle s'engage un ergot (15) prolongeant l'aile (11) du pied (2) de la quille (1).

## Description

La présente invention a pour objet une endo-prothèse totale de genou et des éléments d'essai pour la pose de cette dernière.

Les endo-prothèses totales de genou comprennent une quille comportant d'une part un pied destiné à être ancré dans le tibia et d'autre part une tête en forme de cône morse avec laquelle coopère en blocage l'alésage conique d'un plateau tibial prenant appui sur la partie supérieure aplanie du tibia et recouvert par une pièce d'insert en matière synthétique sur laquelle prend appui, avec possibilité de glissement en rotation, une pièce condylienne fixée en extrémité inférieure du fémur. Les endo-prothèses totales comprennent de plus une pièce rotulienne destinée à être fixée à la face postérieure de la rotule, cette face postérieure étant préalablement arasée dans ce but.

Les différents éléments de la prothèse totale, hormis la pièce d'insert, sont fixés soit au tibia soit au fémur soit à la rotule par du ciment.

La pièce d'insert ainsi que le plateau tibial sont tous deux percés d'un orifice central dans lequel s'engage une vis commune de fixation à la tête conique de la quille.

Les quilles des endo-prothèses de l'art antérieur sont dotées d'un pied constitué par un noyau central duquel rayonnent quatre ailettes d'égales dimensions perpendiculaires deux à deux.
Cette configuration cruciforme régulière qui permet le blocage en rotation de la quille dans le tibia pose problème. En effet, il est nécessaire, lors de l'introduction de la quille dans le tibia, d'éviter tout contact des ailettes du pied avec la zone corticale pour écarter tout risque de fissuration de cette dernière. Pour cette raison, une quille de ce type est nécessairement de longueur réduite et l'orifice qui la reçoit doit être pratiqué dans le tibia de manière décalée vers l'arrière par rapport à l'axe du canal médullaire.
Il est donc impossible d'augmenter la longueur d'une quille du type précité pour s'opposer à l'affaissement du plateau tibial d'une endo-prothèse implantée dans un os fragile résistant mal à la compression.

La pièce d'insert des endo-prothèses de l'art antérieur, de part la nature du matériau qui la constitue est sujette à fluage. Cette pièce d'insert est positionnée sur le plateau tibial de manière précise par un système de tenon et gorge. Le tenon est ménagé sur le plateau tibial tandis que la gorge est pratiquée dans la pièce d'insert ce qui a pour conséquence de réduire sa section et d'augmenter les risques de fluage et donc de déterioration sous l'effet des forces de pression appliquées par la pièce condylienne.

La pièce d'insert et le plateau tibial des prothèses antérieures, comprennent suivant leur bord antérieur une échancrure afin de suivre le contour anatomique de la région correspondante du genou.
Il s'est avéré que l'échancrure est insuffisamment large pour assurer le libre passage au ligament croisé postérieur.

La pièce condylienne doit glisser en rotation sur la surface articulaire ménagée sur la pièce d'insert et ne doit pas rouler sur cette dernière afin d'éviter un effet de tiroir. Pour cette raison, le plan articulaire de la pièce d'insert est incliné, cette inclinaison étant obtenue en donnant une inclinaison à la face arasée du tibia sur laquelle porte le plateau tibial.
Il s'est avéré que cette disposition ne peut à elle seule s'opposer au roulement de la pièce condylienne.

La pièce rotulienne des endo-prothèses de l'art antérieur est de forme ovale et nécessite pour cette raison lors de la pose, une orientation précise obtenue avec difficulté.

La présente invention a pour objet de pallier les inconvénients précédemment évoqués en mettant en oeuvre une endo-prothèse totale de genou comportant une nouvelle quille à ailettes qui d'une part peut être disposée dans le tibia coaxialement au canal médullaire et d'autre part peut présenter une longueur importante sans pour autant venir en contact avec les corticales.

Un autre but de la présente invention est la mise en oeuvre d'une endo-prothèse du genre précité dont les moyens de positionnement de l'insert sur le plateau tibial, outre leur fonction première, ont pour but de renforcer la pièce d'insert.

Un autre but de la présente invention est une endo-prothèse dont la pièce d'insert comporte des premiers aménagements pour offrir un libre passage au ligament croisé postérieur et des seconds aménagements pour s'opposer au roulement de la pièce condylienne sur la surface articulaire qu'elle présente.

Enfin un dernier but de la présente invention est une endo-prothèse dont la pièce rotulienne est conçue de façon à ne pas posséder de sens de pose.

A cet effet, l'endo-prothèse totale de genou selon la présente invention comprenant une quille 1 comportant d'une part un pied 2 destiné à être ancré dans le tibia et d'autre part une tête 3 en forme de cône morse avec laquelle coopère en blocage l'alésage conique 4 d'un plateau tibial 5 prenant appui sur la partie supérieure aplanie du tibia et recevant une pièce d'insert 6 en matière synthétique sur laquelle prend appui avec possibilité de glissement en rotation une pièce condylienne 8 comportant une forme en creux 9 dans laquelle est bloquée la partie inférieure du fémur se caractérise essentiellement en ce que :
- le pied 2 de la quille 1 comporte un noyau central autour duquel sont régulièrement réparties deux ailettes postérieures 10 et une ailette antérieure 11, les deux ailettes postérieures en considérant l'une quelconque section droite étant plus larges que l'ailette antérieure,
- le plateau tibial comprend en saillie sur sa face inférieure un bossage 12 et l'alésage 4 traverse de part en part le plateau et le bossage, lequel bossage comporte d'une part deux ailettes postérieures 13 venant en regard des ailettes postérieures 10 du pied 2 de quille et d'autre part, une fente 14 de positionnement dans laquelle s'engage un ergot 15 prolongeant l'aile antérieure 11 du pied 2 de quille.

La quille grâce à la nouvelle configuration cruciforme que présente son pied peut être diposée dans le canal médullaire sans risque que les ailettes viennent buter contre les zones corticales.

Cette disposition autorise par ailleurs aussi bien l'usage de quilles courtes que de quilles longues.

Selon une autre caractéristique de l'invention, la pièce d'insert 6 présente en regard de la pièce condylienne 8 deux surfaces articulaires 7 sur lesquelles prennent appui respectivement les condyles externe et interne de la pièce condylienne, les dites surfaces articulaires étant séparées l'une de l'autre par un bourrelet médian 6A s'étendant depuis le bord antérieur jusqu'au bord postérieur suivant l'axe médian de symétrie.

Selon une autre caractéristique de l'invention la pièce d'insert 6 et le plateau tibial 5 sont dotés de moyens de positionnement de l'un par rapport à l'autre.

Selon encore une autre caractéristique de l'invention les moyens de positionnement comprennent deux systèmes de gorge 16 et tenon 17 disposés respectivement au droit des deux surfaces articulaires 7, les deux tenons 17 étant enracinés à la face inférieure de l'insert 6 tandis que les gorges 16 sont creusées dans la face supérieure du plateau tibial 5.

Cette caractéristique est propice à améliorer la résistance de la pièce d'insert dans la zone soumise à plus forte charge ce qui diminue les risques de fluage sous fortes pressions exercées par la pièce condylienne.

Selon une autre caractéristique de l'invention, la zone antérieure de chaque face articulaire 7 est relevée vers l'avant tandis que la zone postérieure de chaque face articulaire est relevée vers l'arrière.

Grâce à cette disposition est évité le risque de roulement de la pièce condylienne sur les faces articulaires de la pièce d'insert.

Selon encore une autre caractéristique de l'invention, la pièce rotulienne se présente sous la forme d'une calotte sphérique et comprend en saillie sur sa face plane un pion d'ancrage 26 dans un forage pratiqué dans la rotule, le dit pion présentant un renflement 27 d'extrémité.

Grâce à cette disposition lors de la pose de la pièce rotulienne aucune orientation particulière n'est nécessaire.

D'autres avantages et caractéristiques de l'invention apparaitront à la lecture de la description d'une forme préférée de réalisation donnée à titre d'exemple non limitatif en se référant aux dessins annexés en lesquels :

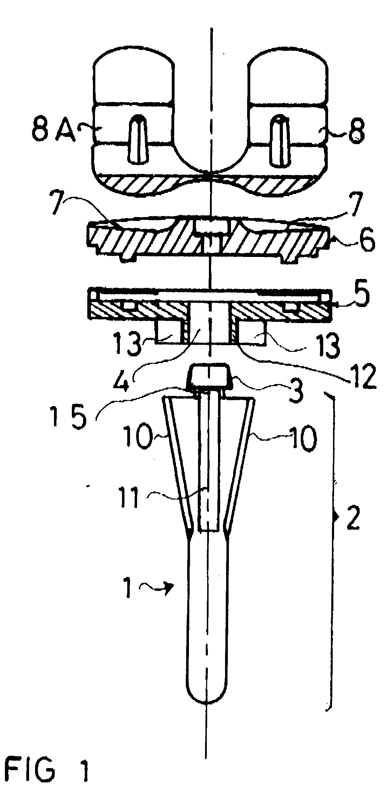

- la figure 1 est une vue d'ensemble en éclaté d'une endo-prothèse selon l'invention,
- la figure 2 est une vue d'une première forme de réalisation de la quille,
- les figures 3 à 6 sont des vues en coupe de la quille suivant respectivement les lignes A/A, B/B, C/C, D/D, de la figure 2,
- la figure 7 est une vue d'une quille suivant une seconde forme de réalisation,
- la figure 8 est une vue de dessous de la quille selon la la figure 7,
- la figure 9 est une vue en coupe selon la ligne E/E de la figure 7,

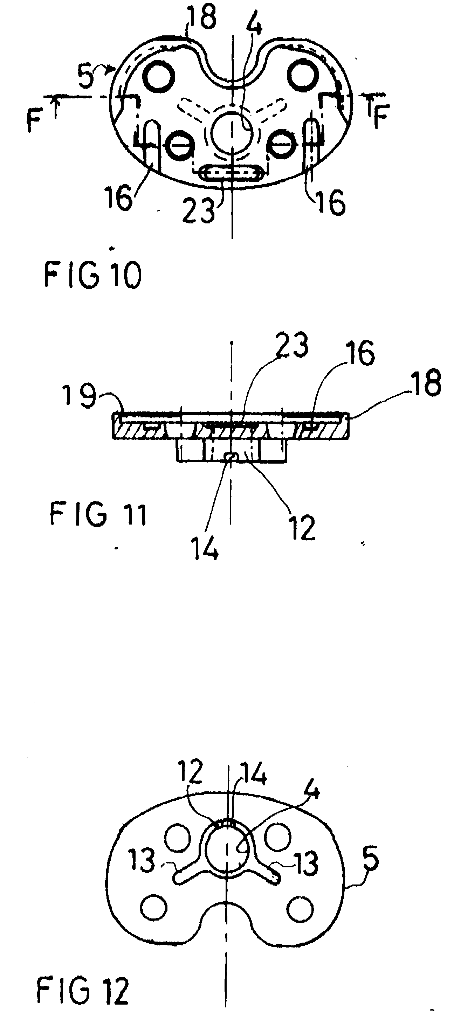

- la figure 10 est une vue de dessus du plateau tibial,
- la figure 11 est une vue en coupe selon la ligne F/F de la figure 10,
- la figure 12 est une vue de dessous du plateau tibial,

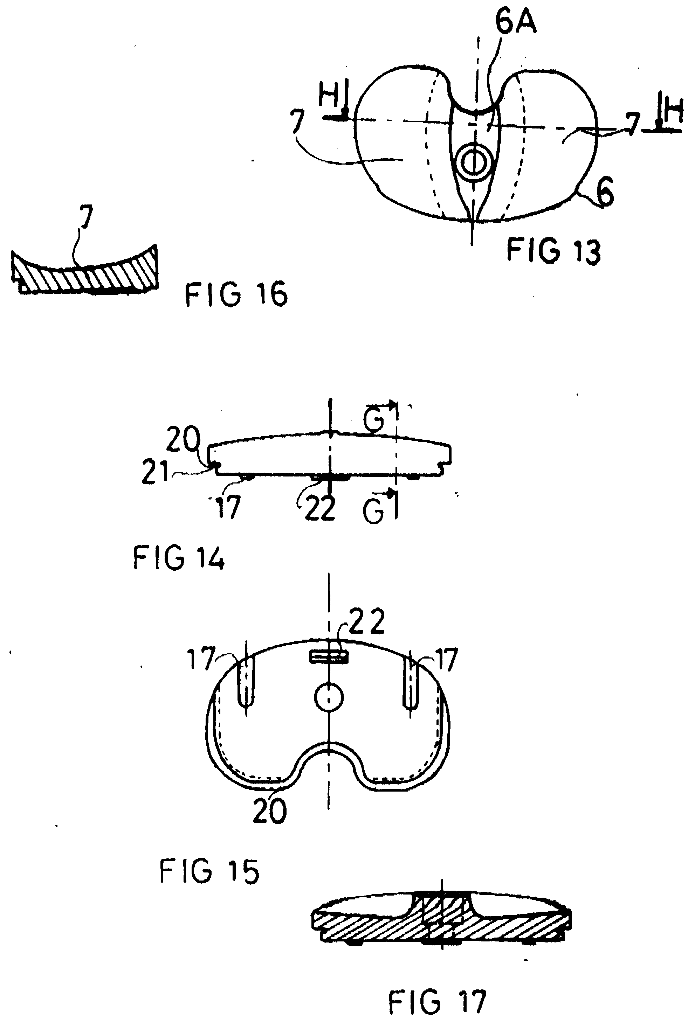

- la figure 13 est une vue de dessus de la pièce d'insert,
- la figure 14 est une vue de face de la pièce d'insert,
- la figure 15 est une vue de dessous de la pièce d'insert,
- la figure 16 est une vue selon la ligne G/G de la figure 14,
- la figure 17 est une vue en coupe selon la ligne H/H de la figure 13,

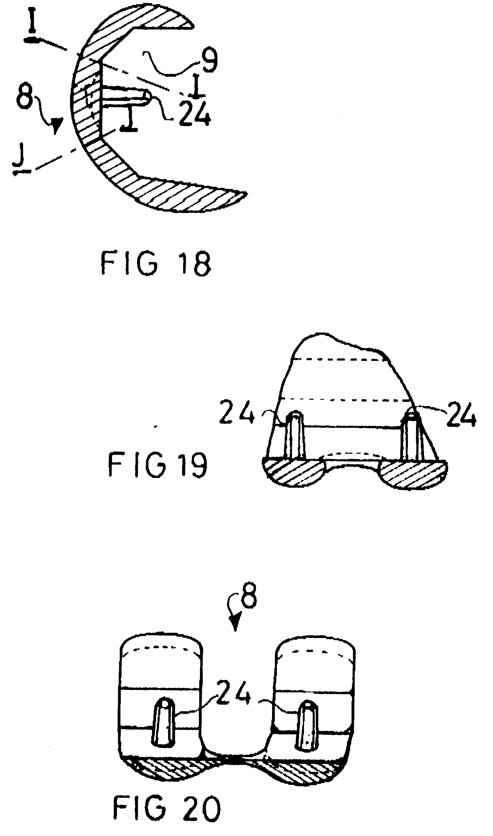

- la figure 18 montre en coupe la pièce condylienne,
- la figure 19 est une vue en coupe selon la ligne I/I de la figure 18,
- la figure 20 est une vue en coupe selon la ligne J/J de la figure 18,

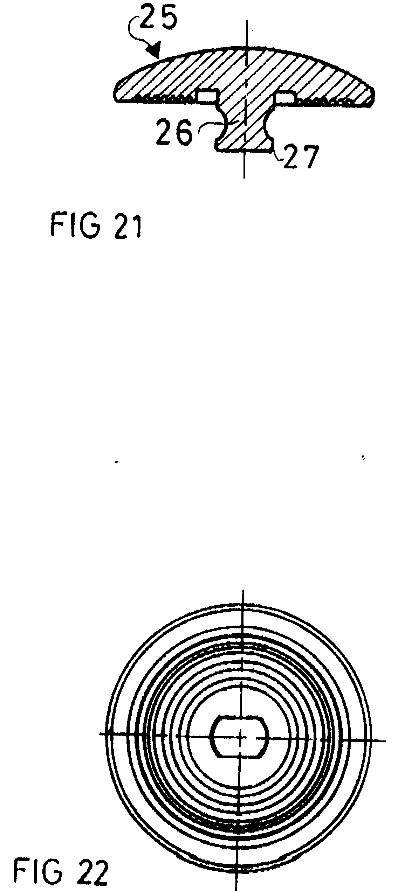

- la figure 21 est une vue en coupe de la rotule,
- la figure 22 est une vue de dessous de la rotule,

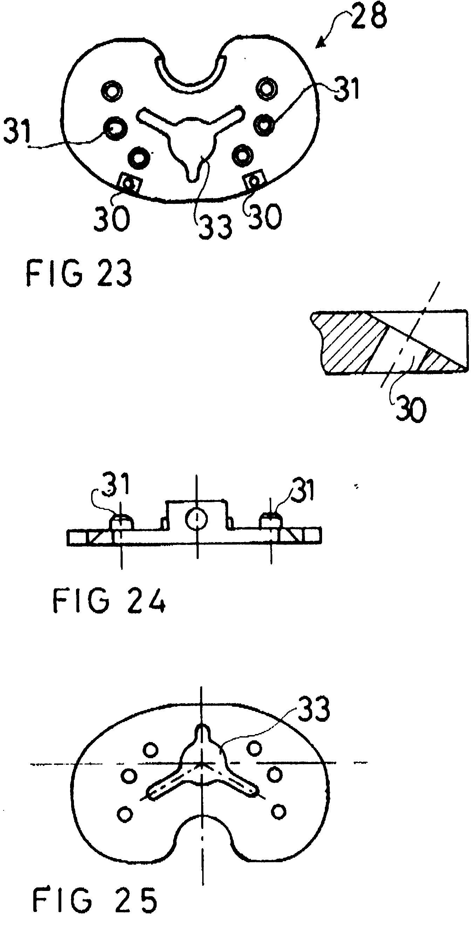

- la figure 23 est une vue de dessus du plateau tibial d'essai,
- la figure 24 est une vue de face du plateau d'essai,
- la figure 25 est une vue de dessous du plateau tibial d'essai,

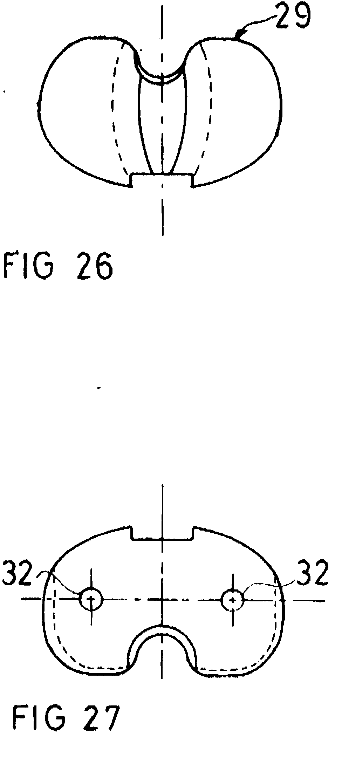

- la figure 26 est une vue de dessus de la pièce d'insert d'essai,
- la figure 27 est une vue de dessous de la pièce d'insert d'essai.

Telle que représentée, l'endo-prothèse totale de genou selon l'invention comprend une quille 1 fixée dans le tibia, un plateau tibial 5 fixé à la quille et au tibia, une pièce d'insert 6 en matière synthétique, fixée sur le plateau tibial, une pièce condylienne 8 fixée en extrémité du fémur et prenant appui sur les surfaces articulaires 7 de la pièce d'insert.

L'endo-prothèse comprend également une pièce rotulienne 25 fixée à la rotule.

La quille 1 comprend un pied 2 destiné à être ancré dans le tibia et une tête 3 en forme de cône morse destinée à coopérer en blocage avec un alésage conique 4 pratiqué dans le plateau tibial.

Le pied 2 de la quille est destiné à venir en léger forcement dans un puit de forme appropriée pratiqué dans le tibia, la forme du puit épousant la forme externe du pied 2.

Le pied 2 s'inscrit dans une surface conique de façon que lors de sa mise en place dans le puit, le ciment de fixation que ce dernier reçoit puisse fluer vers le haut pour se répartir uniformément.

Conformément à l'invention le pied 2 de la quille 1 comporte un noyau central 2A autour duquel sont régulièrement réparties deux ailettes postérieures 10 et une ailette antérieure 11.

Chaque ailette est séparée de l'ailette contigue par un angle de 120°.

En considérant l'une quelconque section droite du pied on peut remarquer que les ailettes postérieures 10 sont plus larges que l'ailette antérieure 11.

Cette configuration permet la mise en place de la quille dans un puit pratiqué dans le tibia suivant l'axe médullaire de ce dernier sans crainte de contact avec la face interne de la zone corticale de l'os.

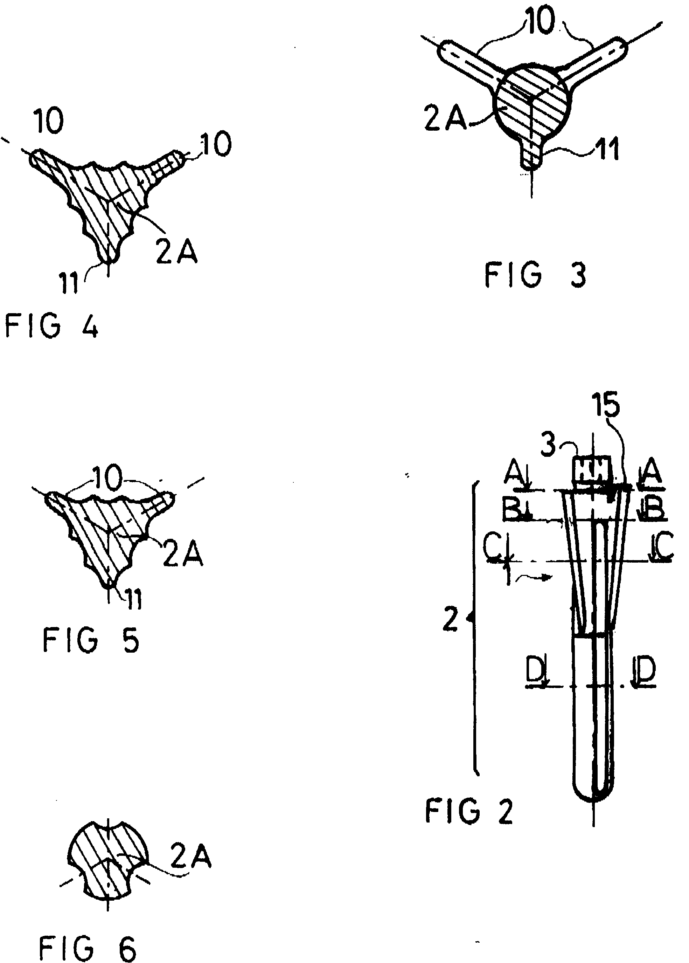

En figures 2 à 6 est représentée une quille suivant une première forme de réalisation. Cette quille avec un pied de longueur importante est destinée à être mise en place dans un os fragile.

Cette quille comprend un noyau épais prolongé en dessous des ailettes par une partie cylindrique arrondie en extrémité et de longueur plus ou moins importante.

En figure 2, on a représenté une quille dont la partie cylindrique du pied présente une longueur importante mais en variante cette longueur pourra être réduite.

La quille 1 selon cette dernière variante pourra être implantée dans un os peu fragile.

Entre chaque ailette, le pied 2 de cette quille est pourvu d'une rainure longitudinale, laquelle couvre par ailleurs toute la longueur de la partie cylindrique du pied.

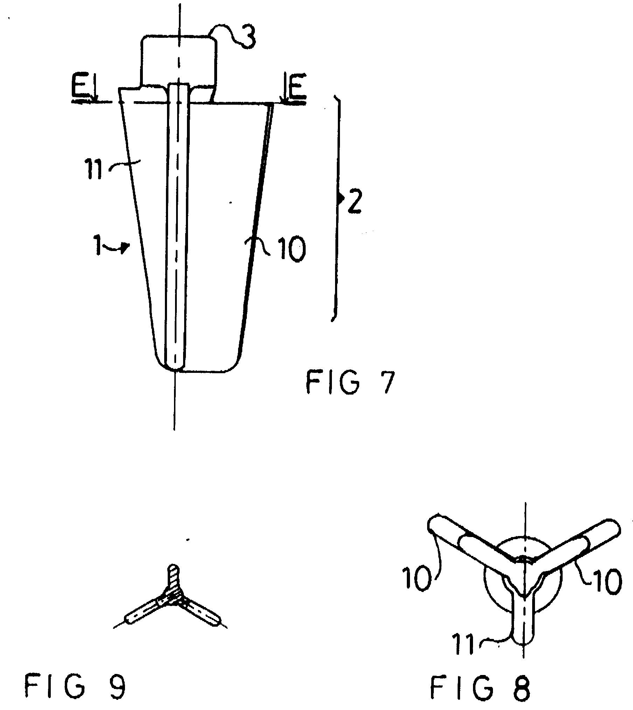

En figures 7 à 9 est représentée une quille suivant une seconde forme de réalisation. cette quille 1 avec un pied 2 de faible longueur est destinée a être implantée dans un os peu fragile et les ailettes 10, 11 occupent toute la longueur du pied 2.

Le plateau tibial 5 prend appui sur une face plane formée en extrémité du tibia par arasement. Comme connu une couche de ciment peut être interposée entre le plateau tibial et la face plane du tibia. Pour une meilleure adhérence et une meilleure integrétion de l'os ou du ciment la face inférieure du plateau tibial est revêtue d'une couche de matière poreuse. Il y a lieu de noter que le plateau tibial 5 peut être posé sans ciment.
Le plateau tibial 5 épouse le contour du plateau tibial anatomique.

Le plateau tibial 5 comprend un bord antérieur arrondi deux bords latéraux également arrondis et un bord postérieur pourvu d'une échancrure de taille suffisamment importante pour assurer le libre passage au ligament croisé postérieur. Le plateau tibial 5 possède par ailleurs un axe médian de symétrie sécant aux bords antérieurs et postérieurs, l'échancrure postérieure du plateau tibial 5 étant formée de part et d'autre de l'axe médian et admet ce dernier pour axe de symétrie.

Par ailleurs, le plateau tibial comprend en saillie sur sa face inférieure un bossage 12 et l'alésage 4 pratiqué dans le dit plateau traverse de part en part ce dernier ainsi que le bossage. La conicité de l'alésage 4 ainsi que la conicité de la tête 3 sont propres d'une part à faciliter la mise en place du plateau 5 sur la quille et d'autre part à assurer un blocage par adhérence du plateau tibial sur la tête de la quille.

Il est nécessaire que le plateau tibial soit positionné avec précision par rapport à la quille. Dans ce but, le bossage 12 est pourvu d'une fente radiale 14 de positionnement dans laquelle s'engage un ergot 15 porté par l'aile antérieure 11 du pied de quille, le dit ergot 15 étant en saillie vers le haut et dans le prolongement de la dite aile 11.

L'ergot 15 et la fente 11 réalisent une liaison par obstacle liant en rotation le plateau tibial par rapport à la quille.
Le bossage 12 du plateau tibial 5 comporte deux ailettes postérieures 13 venant en regard des ailettes postérieures 10 du pied 2 de la quille au contact de ces dernières ou bien en écartement.

Le plateau tibial 5 comprend quatre perçages de passage de vis de fixation au tibia, dont deux sont disposés en zone postérieure et les deux autres en zone antérieure. Les orifices postérieurs sont disposés au plus près du bord postérieur et de part et d'autre de l'échancrure de passage du ligament croisé postérieur.

La tête de chaque vis de fixation au tibia est en forme de calotte sphérique, ce qui autorise une disposition oblique dans son perçage. Cette disposition, notamment, pour les vis postérieures, autorise un ancrage de ces dernières dans la corticale postérieure si nécessaire.

Le contour de la pièce d'insert 6 suit le contour du plateau tibial 5 et la dite pièce d'insert 6, tout comme le plateau tibial 5, présente un bord antérieur arrondi, deux bords latéraux et un bord postérieur dans lequel est creusée une échancrure de taille relativement importante pour assurer le libre passage au ligament croisé postérieur. Cette pièce d'insert, tout comme le plateau tibial, présente un axe médian de symétrie, sécant aux bords antérieur et postérieur, l'échancrure se développant de manière symétrique de part et d'autre du dit axe médian.

La pièce d'insert 6 présente en regard de la pièce condylienne 8 deux surfaces articulaires 7 sur lesquelles prennent appui respectivement les condyles externe et interne de la pièce condylienne 8, les dites surfaces articulaires 7 étant séparées l'une de l'autre par un bourrelet médian 6A s'étendant depuis le bord antérieur jusqu'au bord postérieur.

La pièce d'insert 6 vient recouvrir parfaitement le plateau tibial 5 et est maintenue contre ledit plateau par une vis de blocage engagée dans un perçage pratiqué dans le bourrelet 6A, au droit de l'alesage conique 4. La vis coopère en vissage avec un taraudage axial borgne pratiqué dans la tête 3.
Le perçage est aménagé en lamage de façon à noyer totalement la tête de la vis.

De façon que la pièce d'insert 6 soit maintenue parfaitement centrée par rapport au plateau tibial 5, sont prévus des moyens de positionnement de l'un par rapport à l'autre
Ces moyens comprennent deux systèmes de gorge 16 et tenon 17 disposés respectivement au droit des deux surfaces articulaires 7, les deux tenons 17 étant enracinés à la face inférieure de l'insert 6 tandis que les gorges 16 sont creusées dans la face supérieure du plateau tibial 5.
Les tenons 17 constituent, au droit des surfaces articulaires 7, sous la pièce d'insert 6 des nervures de renfort propices à améliorer la résistance au fluage.
De préférence, les gorges 16 et les tenons 17 s'étendent parallèlement à l'axe médian de symétrie de leur pièce 6, 7 respective et ce depuis le bord antérieur de leur dite pièce. Cette disposition autorise la mise en place de la pièce d'insert 6 sur le plateau 5 par mouvement de glissement suivant l'axe médian de symétrie.

Afin de complèter le positionnement de la pièce d'insert 6 sur le plateau tibial 5 est prévue une paroi de butée 18 ménagée en saillie sur la face supérieure du plateau tibial 5 suivant les côtés latéraux et postérieur de ce dernier, contre laquelle vient buter les bords latéraux et postérieur de la pièce d'insert 6. La pièce d'insert 6 comporte suivant ses côtés latéraux et postérieur une paroi de recouvrement 20 destinée à venir recouvrir la rive supérieure de la paroi de butée 18. De surcroit, cette paroi de butée 18, suivant au moins ses côtés latéraux comporte un rebord 19 en surplomb sur la face supérieure du plateau 5, recouvert par la paroi 20 et destiné à pénétrer dans une fente 21 creusée dans la rive verticale de la pièce d'insert correspondant aux côtés latéraux.
Grâce à cette disposition, la pièce d'insert 6, avant fixation par vissage, ne pourra s'écarter du plateau tibial 5. Préférentiellement, la fente 21 et le rebord 19 sont prolongés suivant le côté postérieur de leur pièce respective jusqu'à la naissance de l'échancrure.

Il est également prévu comme moyen de positionnement un ergot 22 allongé en saillie sur la face inférieure de la pièce d'insert 6 et une gorge 23 creusée dans la face supérieure du plateau tibial, ledit ergot 22 et la dite gorge 23 étant pratiqués perpendiculairement à l'axe médian de symétrie à leur pièce respective dans la zone antérieure de cette dernière.
De façon à faciliter son introduction dans la gorge 23, l'ergot 22 sera pourvu d'un chanfrein.

Selon une forme préférée de réalisation, la zone antérieure de chaque face articulaire 7 est relevée vers l'avant tandis que la zone postérieure de chaque face articulaire est relevée vers l'arrière.
Cette disposition évite le roulement de la pièce condylienne sur les surfaces articulaires 7.
Selon la forme préférée de réalisation, les zones antérieures et postérieures de chaque face articulaire 7 se relèvent chacune en arc de circonférence de cercle.

En variante, la zone postérieure de chaque face articulaire sera plane.

Chaque surface articulaire 7 de la pièce d'insert 6 est en pente vers le bas depuis le bord latéral vers le bourrelet médian 6A.

La pièce condylienne 8 présente une forme en creux 9 dotée de cinq facettes. Par la forme en creux 9, la pièce condylienne vient coiffer l'extrémité inférieure du fémur à laquelle par arasement est donnée la forme interne de la pièce condylienne 8. Entre la forme condylienne et l'extrémité du fémur peut être interposée une couche de ciment de liaison. Par ailleurs, dans la forme en creux, la pièce condylienne est dotée de deux pions coniques 24 venant s'ancrer dans deux perçages pratiqués en extrémité du fémur.

La pièce rotulienne 25 constituée en matière synthétique se présente sous la forme d'une calotte sphérique et comprend en saillie sur sa face plane un pion d'ancrage 26 destiné à pénétrer dans un forage de la rotule. Le pion présente un renflement 27 d'extrémité.

Grâce à cette disposition, la pièce rotulienne n'a nul besoin d'être positionnée suivant une orientation privilégiée.
La face plane de la calotte sphérique est pourvue de saillies et creux circulaires pour améliorer l'adhérence du ciment de liaison à la rotule.
Le forage pratiqué dans la rotule reçoit du ciment de liaison. Le renflement 27 que présente le pion 26 en extrémité a pour but de constituer organe de rétention de la pièce rotulienne contre la rotule.

L'endo-prothèse telle que décrite est associée à des pièces d'essai pour la détermination de la taille du plateau tibial 5 et de la pièce d'insert 6.
Ces pièces d'essai sont constituées par un plateau tibial d'essai 28 et une pièce d'insert d'essai 29, comportant un moyen de positionnement et d'encliquetage de l'un par rapport à l'autre, le plateau d'essai 28 comportant par ailleurs en périphérie au moins deux orifices inclinés 30 destinés à recevoir un clou de fixation au tibia.

Selon la forme préférée de réalisation, le moyen de positionnement et d'encliquetage est constitué par exemple par deux pions 31 en saillie sur le plateau tibial et par deux orifices 32 pratiqués dans l'épaisseur de la pièce d'insert d'essai dans lesquels s'engagent les pions 31. Les deux orifices 32 de l'insert d'essai 29 pourront avantageusement traverser de part en part le dit insert pour améliorer le nettoyage. Ces deux orifices 32 pourront également être borgnes.

Le chirurgien dispose de plusieurs plateaux d'essai et de plusieurs pièces d'insert d'essai correspondant aux différentes tailles. Après mise en place de la pièce condylienne, il choisit le plateau d'essai et la pièce d'insert d'essai qui semblent le mieux convenir et procède à leur mise en place sur le tibia.
Après examen de la répartition des contraintes dans le genou lors de la flexion et éventuellement après plusieurs essais successifs, il détermine la taille du plateau tibial 5 et de la pièce d'insert 6. Il procède alors au retrait de la pièce d'insert de façon a dégager un orifice cruciforme 33 traversant de part en part le plateau tibial d'essai.
Cet orifice cruciforme sert de canon de guidage à un outil de forage dans le tibia, du puit destiné à recevoir le pied 2 de quille. Ensuite, il procèdera au retrait du plateau tibial d'essai et à la mise en place des éléments de l'endo- prothèse.
Il y a lieu de noter que la pièce d'insert d'essai 29 pourra comporter également une paroi de recouvrement et une fente conformes à la paroi 20 et à la fente 21 de la pièce d'insert 6 afin de permettre d'effectuer un essai sur le plateau tibial 5 définitif.

## Revendications

1. Endo-prothèse totale de genou comprenant une quille (1) comportant d'une part un pied (2) destiné à être ancré dans le tibia et d'autre part une tête (3) en forme de cône morse avec laquelle coopère en blocage l'alésage conique (4) d'un plateau tibial (5) prenant appui sur la partie supérieure aplanie du tibia et recevant une pièce d'insert (6) en matière synthétique comportant des surfaces articulaires (7) sur laquelle prend appui, avec possibilité de glissement en rotation une pièce condylienne (8) comportant une forme en creux (9) dans laquelle est bloquée la partie inférieure du fémur caractérisée en ce que :
- le pied (2) de la quille (1) comporte un noyau (2A) central autour duquel sont régulièrement réparties deux ailettes postérieures (10) et une ailette antérieure (11) , les deux ailettes postérieures (10) en considérant l'une quelconque section droite étant plus large que l'ailette antérieure (11),
- le plateau tibial (5) comprend en saillie sur sa face inférieure un bossage (12), et l'alésage (4) traverse de part en part le plateau (5) et le bossage (12), lequel bossage comporte d'une part deux ailettes postérieures (13) venant en regard des ailettes postérieures (10) du pied (2) de quille, et d'autre part, une fente (14) de positionnement dans laquelle s'engage un ergot (15) prolongeant l'aile antérieure (11) du pied (2) de quille.

2. Endo-prothèse selon la revendication 1 caractérisée en ce que la pièce d'insert (6) présente en regard de la pièce condylienne (8) deux surfaces articulaires (7) sur lesquelles prennent appui respectivement les condyles externe et interne de la pièce condylienne (8), les dites surfaces articulaires (7) étant séparées l'une de l'autre par un bourrelet médian (6A) s'étendant depuis le bord antérieur jusqu'au bord postérieur suivant l'axe médian de symétrie.

3. Endo-prothèse selon la revendication 1 caractérisée en ce que la pièce d'insert (6) et le plateau tibial (5) sont dotés de moyens de positionnement de l'un par rapport à l'autre.

4. Endo-prothèse selon la revendication 3 caractérisée en ce que les moyens de positionnement comprennent deux systèmes de gorge (16) et tenon (17) disposés respectivement au droit des deux surfaces articulaires (7), les deux tenons (17) étant enracinés à la face inférieure de l'insert (6) tandis que les gorges (16) sont creusées dans la face supérieure du plateau tibial (5).

5. Endo-prothèse selon la revendication 4 caractérisée en ce que les gorges (16) et les tenons (17) s'étendent parallèlement à l'axe médian de symétrie de leur pièce respective et ce, depuis le bord antérieur de leur dite pièce.

6. Endo-prothèse selon la revendication 3 caractérisée en ce que les moyens de positionnement comprennent également :
- une paroi de butée (18) ménagée en saillie sur la face supérieure du plateau tibial (5) suivant les côtés latéraux et postérieur de ce dernier et comportant suivant au moins les côtés latéraux un rebord (19) venant en surplomb sur la face supérieure du plateau tibial,
- une paroi de recouvrement (20) ménagée suivant les côtés latéraux et postérieur de la pièce d'insert (6) destinée à venir recouvrir la paroi (18) de butée du plateau tibial contre laquelle vient buter la pièce d'insert (6) par ses côtés latéraux et postérieur, la dite pièce d'insert comportant par ailleurs suivant au moins ses côtés latéraux une fente (21) dans laquelle s'engage le rebord du plateau tibial.

7. Endo-prothèse selon la revendication 3 caractérisée en ce que les moyens de positionnement comprennent également un ergot (22) allongé en saillie sur la face inférieure de la paroi d'insert et une gorge (23) creusée dans la face supérieure du plateau tibial, ledit ergot (22) et la dite gorge (23) étant pratiqués perpendiculairement à l'axe médian de symétrie de leur pièce respective dans la zone antérieure de cette dernière.

8. Endo-prothèse selon la revendication 2 caractérisée en ce que la zone antérieure de chaque face articulaire (7) est relevée vers l'avant tandis que la zone postérieure de chaque face articulaire est relevée vers l'arrière.

9. Endo-prothèse selon la revendication 8 caractérisée en ce que les zones antérieure et postérieure de chaque face articulaire (7) se relèvent chacune en arc de circonférence de cercle.

10. Endo-prothèse selon la revendication 2 caractérisée en ce que chaque face articulaire (7) de la pièce d'insert (6) est en pente vers le bas depuis le bord latéral vers le bourrelet médian (6A).

11. Endo-prothèse selon la revendication 1 caractérisée en ce que la pièce rotulienne se présente sous la forme d'une calotte sphérique et comprend en saillie sur sa face plane un pion d'ancrage (26) dans un forage de la rotule, le dit pion présentant un renflement (27) d'extrémité.

12. Pièces d'essai pour la pose d'une endo-prothèse selon l'une quelconque des revendications précédentes caractérisées en ce qu'elles sont constituées par un plateau tibial d'essai (28) et une pièce d'insert d'essai (29) comportant un moyen de positionnement et d'encliquetage de l'un par rapport à l'autre, le plateau d'essai (28) comportant par ailleurs en périphérie au moins deux orifices inclinés (30) destinés à recevoir un clou de fixation au tibia.
